Europäisches Patentamt

(19) European Patent Office

Office européen des brevets

(11) Publication number: **0 041 114**
**B1**

(12) **EUROPEAN PATENT SPECIFICATION**

(45) Date of publication of patent specification: **05.11.86**

(21) Application number: **81102533.7**

(22) Date of filing: **03.04.81**

(51) Int. Cl.⁴: **A 61 K 9/20,** A 23 K 1/17

(54) Process for the preparation of medicated animal feed supplement.

(30) Priority: **08.05.80 US 147805**

(43) Date of publication of application:
**09.12.81 Bulletin 81/49**

(45) Publication of the grant of the patent:
**05.11.86 Bulletin 86/45**

(84) Designated Contracting States:
**CH DE FR GB IT LI NL**

(56) References cited:
**FR-A-2 318 593**
**US-A-3 015 611**
**US-A-3 531 563**
**US-A-4 048 268**

The file contains technical information
submitted after the application was filed and
not included in this specification

(73) Proprietor: **AMERICAN CYANAMID COMPANY**
**1937 West Main Street P.O. Box 60**
**Stamford Connecticut 06904 (US)**

(72) Inventor: **Klothen, Irving**
**215 Mt. Lucas Road**
**Princeton New Jersey 08540 (US)**

(74) Representative: **Wächtershäuser, Günter, Dr.**
**Tal 29**
**D-8000 München 2 (DE)**

Courier Press, Leamington Spa, England.

# 0 041 114

## Description

The invention is a process for preparing medicated animal feeds, feed additives, feed supplements or feed premixes, characterized by using in the process an antibiotic, a sulfa drug, penicillin, monensin, tylosin or lasalocid or a mixture of these drugs by either

(a) compacting the drug or mixture of drugs or

(b) compacting a blend of the drug or mixture of drugs blended with a compressible and pharmaceutically acceptable inert diluent or diluent mixture (in the absence of added water) with sufficient force to obtain a

(1) compacted drug
(2) compacted mixture of drugs
(3) compacted blend of drug and diluent or diluents or
(4) compacted blend of a mixture of drugs and a diluent or diluents

of at least 10 kg to 30 kg hardness on a Stokes hardness tester and grinding and sifting the compacted material to a particle size range of 2.0 to 0.099 mm to prevent excessive build-up, deposition, retention and dusting of the drug or mixture of drugs in the process.

Feed mills in which medicated animal feed premixes, supplements, concentrates or finished medicated animal feeds are prepared, have a tendency to retain some of the finely divided drugs due to electrostatic adhesion, dusting or some other phenomenon associated with the day-to-day operation of such installations. The retention of drugs within the mill is obviously undesired since it may, and usually does, result in the contamination of subsequent batches of nonmedicated animal feed or supplement prepared in said mill.

It is, therefore, the objective of the present invention to provide a method for the prevention of excessive build up, deposition, retention and/or dusting of a drug or mixture of drugs in a feed mill or in any other equipment used for the preparation of the above animal feed products, which would result in the contamination of same with said drugs while traversing said mill or other equipment in the course of being prepared; comprising: a process of compacting a drug or a mixture of drugs, optionally blended with a compressible, nutritionally acceptable and pharmaceutically inert diluent or diluent mixture, with sufficient force to obtain a compacted material of at least 10 kg to 30 kg hardness on a Stokes hardness tester and of a particle size range of 10 to 150 mesh.

The particles of the premixes obtained by the process of the present invention are designed to be of a size range large enough not to adhere to processing equipment surfaces (or to packaging surfaces) as for instance by electrostatic adhesion, or to be carried away in a dust collecting stream, and yet at the same time to be of an adequately small size range so that a statistically sufficient number of particles are present in the finished feed premix to assure uniform distribution throughout the batch of feed into which it is blended to achieve the desired drug concentration.

In general, conventional medicated animal feed premixes are prepared by blending finely powdered drugs (crude or pure) and/or micronutrients with pharmaceutically and nutritionally acceptable diluents or carriers to obtain drug concentrations in the premixes so that convenient quantities, e.g. 0.5, 1 or 2 lb (or kg) per ton of feed can be weighed out by the feed formulators. Unfortunately, in such premixes, the drug particles remain discrete and thus their distribution pattern is independent of the other ingredients of the premix. When blended with animal feed, wide particle size ranges or unexpected agglomeration may change this pattern from the one desired.

The difficulties encountered by using fine powders in medicated animal feed premixes has been recognized, and a number of attempts have been made to overcome them. Thus, such formulations have been aggregated on to the surface of coarse carriers by the use of suitable binders or sticking agents; while other attempts relate to the preparation of wet aggregates followed by granulation and subsequent drying of the granules. Unfortunately, these processes are less than satisfactory. The former process yields aggregates which do not adhere permanently, drying and attrition during storage and handling releases free, fine drug particles which again become subject to segregation, adhesion to surfaces and to removal as dust. The latter process is generally too costly for this type of application and also, because of the frequent use of water, causes stability problems with many drugs.

It is now found that by the process and composition of my invention, granular, medicated animal feed premixes can be prepared from antibiotic, sulfur drug, penicillin, monensin, tylosin or lasalocid acid or combination of these drugs by combining these with the necessary amount of a compressible, inert and pharmaceutically and nutritionally acceptable diluent, such as whey solids, in the absence of added water or any other liquid binder, followed by blending, compacting and granulating the composition under sufficiently mild conditions so as to avoid adiabatic overheating (caused by some extrusion processes) of the mixtures and thus prevent or minimize thermal decomposition of the components. Furthermore, since thorough blending precedes the compacting and granulation steps, the finished granules are of relatively uniform potency throughout, and having been compacted under pressure prior to granulation, the granules possess better resistance to crushing, crumbling and surface abrasion during storage and during blending with feed.

2

0 041 114

Consequently, more uniform distribution of drugs in the feed can be expected; and, in general, the feed premixes prepared by the process of the invention are useful for the distribution of any drug or microingredient in feedstuffs.

The process of the present invention is especially useful for the preparation of medicated premixes containing pure drugs, since these are, as a general rule, used in very small amounts and, in addition, are usually very fine in particle size and thus tend to be subject to various types of segregation from the feed matrix. Specifically, as stated above, the particulated compositions prepared by the process of this invention are of a size range so that adhering, and thus retention of the product in the blending conveying and compacting equipment, is minimized. Retention in said equipment would normally lead to the phenomenon known as drug carry-over or cross-contamination.

Medicated feed premixes prepared by the process of the present invention comprise: about 5% by weight to about 65% by weight of composition, a finely divided drug or a mixture of drugs hereinbelow defined and described in detail and a compressible, nutritionally acceptable and pharmaceutically inert diluent or diluent mixture in amounts sufficient to total said composition to 100%.

Conveniently, by the process and composition of the invention, a medicated feed premix can be prepared by admixing an antibiotic such as Avoparcin, Bacitracin, Bambermycin, Griseofulvin, Hygromycin B, Lincomycin, Monensin, Neomycin, Nystatin, Oleandomycin, Streptomycin, Tylosin and salts thereof, Virginiamycin or a tetracycline antibiotic such as tetracycline, chlortetracycline, oxytetracycline or dimethylchlortetracycline and mixtures thereof, either as the pure drugs or the fermentation solids containing the solids, a sulfa drug such as sulfamethazine, sulfaethoxypyridazine, sulfadimethoxine, sulfaquinoxaline, sulfathiazole or other suitable sulfa drugs, carbadox, thiabendazole, procaine penicillin G or other stable penicillin salts, an inert diluent which has been found to compact well, such as whey solids, lactose, sucrose, cellulose, ground oyster shells, calcium carbonate, dicalcium phosphate or mixtures thereof, and preferably whey solids. The mixture thus obtained is then thoroughly blended and fed into a set of compacting rolls which are under hydraulic pressure of from 90 kg cm$^{-2}$ to 212 kg cm$^{-2}$. The compacted material emerges from the rolls in various shapes such as briquettes, ribbed sheets and the like depending on the type of compacting rolls chosen. The emerging solid shapes possess a Stokes hardness range of from 10 kg to 30 kg in contrast to conventional granulated feed premixes having a hardness range of from about 5 kg to 8 kg. Finally, the material thus obtained is ground or fragmented by suitable means to the desired particle size range, usually from 10/40 to 40/150 mesh (2 mm to 0,099 mm).

The above description of the process of the invention suggests compositions comprising at least one of each of the antibiotics selected from the group defined above, a sulfa drug, carbadox or thiabendazole and a penicillin antibiotic as the active components of said composition. It should be clearly understood, however, that the above process can be used successfully to prepare medicated feed premixes having the above described desirable characteristics, but containing only one of the above described drugs, especially sulfa drugs.

Thus, for instance, 25% by weight to about 35% by weight of composition of chlortetracycline fermentation solids comprising: chlortetracycline calcium complex, mycelial solids, nutrient residues and filteraid, and containing 10% by weight to about 25% by weight of chlortetracycline; 2% to 10% by weight of formulation of sulfamethazine, 2% to 10% by weight of procaine penicillin G and sufficient amount of whey solids to total the mixture to 100%, are mixed. The above whey solids have the following composition:

| Lactose, % | 50—67.7 |
| Protein, % | 12.1—17.2 |
| Minerals, % | 9.6—15.6 |
| Fat, % | 0.75—1.0 |
| Moisture, % | 4.5—6.0 |
| Fiber, % | — |
| Calcium, % | 0.8—1.5 |
| Phosphorus, % | 0.7—1.0 |
| Riboflavin, mg/kg | 24.25—52.91 |
| Niacin, mg/kg | 11.02—17.64 |
| Thiamine, mg/kg | 3.97—4.85 |

3

| | |
|---|---|
| Panthotenic acid, mg/kg | 48.5—74.96 |
| Choline, mg/kg | 2,425.08—4,188.78 |
| Pyridoxine, mg/kg | 2.87—5.29 |
| Vitamin $B_{12}$, mmg/kg | 26.1—33.07 |
| Folic Acid, mmg/kg | 132.28—253.53 |
| Biotin, mmg/kg | 352.74—595.25 |

Thus, for instance, the supplement or premixes comprises 16% by weight to 41% by weight of composition of chlortetracycline fermentation solids containing from 12% by weight to 27% by weight of chlortetracycline; 2% by weight to 6% by weight of composition of sulfamethazine; 2% by weight to 6% by weight of composition of procaine penicillin G; wherein the compacted material has a particle size range of 1.19 to 0.250 mm.

The supplement or premix could also comprise: 32% by weight to 82% by weight of composition of chlortetracycline fermentation solids containing from 12% by weight to 27% by weight of chlortetracycline; 8% by weight to 13% by weight of composition of sulfamethazine; 7% by weight to 11% by weight of composition of procaine penicillin G; wherein the compacted material has a particle size range of 1.19 to 0.250 mm.

The mixture is then thoroughly blended and fed into a pair of compacting rolls which are under a hydraulic pressure of from about 90 kg cm$^{-2}$ to about 212 kg cm$^{-2}$, and finally, the shaped, compacted material is ground and sifted to a particle size range of from 2.0 mm to 0.099 mm, and preferably 1.19 mm to 0.250 mm.

Similarly, by the above procedure, medicated feed premixes are prepared, comprising 10% to 100% by weight of composition of a sulfa drug selected from sulfamethazine, sulfaethoxypyridazine, sulfathiazole or other suitable sulfa drugs and mixtures thereof; and when the amount of said drugs is less than 100% an inert diluent or mixture of diluents, selected from the group hereinabove described, in amounts sufficient to total said premix to 100%.

Additives such as antistatic agents, coating agents and drying agents may be incorporated in the premixes, if so desired.

The medicated premixes prepared by the process of the invention are designed to allow incorporation of about 100 g of tetracycline antibiotic, of about 100 g of sulfa drug and of about 50 g penicillin per ton of feed.

The process can easily be changed to accommodate any other desired drug concentration by varying the concentration of the drug in the feed premix or by changing the accepted particle size range so that an adequate number of particles of compacted premix per ton of feed are introduced to assure statistically uniform distribution.

The following non-limiting Examples are provided to further illustrate the invention.

Example 1
Preparation of medicated granular feed premixes having the composition of the invention
Medicated feed premix batches are prepared, having the composition and size as given in Table I below.

The components are mixed and blended for 10 minutes in a ribbon blender. The blended composition is then fed to 2.5 cm (1″) wide briquetting rolls of 12.7 cm (5″ diameter) at a predetermined pressure and rate to yield briquettes or ribbons having a range of desired hardness. These compacts are then ground and sieved to 0.69/0.250 mm and <0.280 mm fractions. Table II below lists the number of batches prepared, the compacting roll pressures used and the degree of hardness achieved.

For comparison, a sample of a standard, medicated premix having the same composition, except for the diluent is compacted, and the pertinent data entered as No. 14 in Table II below, wherein it can be clearly seen that the composition of the present invention yields much harder material than comparable, conventional blends.

4

TABLE I
Composition and size of medicated feed premixes of the present invention

| Component of composition | % Purity | % by weight of composition | g per batch | Drug | | |
|---|---|---|---|---|---|---|
| | | | | g=real per batch | g/kg of formulation | g/lb of formulation |
| Chlortetracycline fermentation | 15.4 | 32 | 1,600 | 249.9 | 49.4 | 22.4 |
| Sulfamethazine | 100 | 4.6 | 230 | 230 | 46.0 | 20.9 |
| Procaine Penicillin G | 60 | 4.0 | 200 | 120 | 24.0 | 10.9 |
| Whey solids q.s. ad 100 | | 59.4 | 2,970 | | | |
| Totals: | | 100.0 | 500 | | | |

# 0 041 114

| No. | Feed screw pressure kg cm$^{-2}$ | Stokes hardness kg |
|---|---|---|
| 1 | 94.5 | 25.5 |
| 2 | 94.5 | 25.9 |
| 3 | 122.5 | 29.6 |
| 4 | 154.0 | 26.3 |
| 5 | 189.0 | 30.0 |
| 6 | 189.0 | 28.5 |
| 7 | 189.0 | 25.5 |
| 9 | 189.0 | 23.3 |
| 10 | 189.0 | 19 |
| 11 | 210.0 | 15.8 |
| 12 | 210.0 | 26.5 |
| 13 | 94.5 | 22.5 |
| 14 | 94.5 | 7.5 |

Example 2

Large scale preparation of medicated, granular feed premix having the composition of the invention

By the method of Example 1, chlortetracycline fermentation solids (18.94% pure; 4,732 g as is material=26% by weight of composition), sulfamethazine (100% pure; 840 g=4.63% by weight of composition), procaine penicillin G (60% pure; 734 g=4.05% by weight of composition) and whey solids (11,837 g=65.52% by weight of composition) are blended and fed at a rate of 800 g/minutes and at 129.5 g kg cm$^{-2}$ pressure to 5 cm (2″) wide 12.7 cm (5″) diameter corrugated rolls to be compacted to the desired degree of hardness. Finally, the material is granulated to the desired mesh size range, 20 to 60 mesh.

The thus prepared premix provides:

| Ingredient | g/kg | g/lb |
|---|---|---|
| Chlortetracycline | 49.4 | 22.4 |
| Sulfamethazine | 46.3 | 21.0 |
| Procaine Penicillin G | 24.25 | 11.0 |

By the above method, excepting that calcium carbonate is substituted for whey solids, a medicated granular feed premix is prepared comprising:

| Ingredient | Purity | % by weight of composition |
|---|---|---|
| Chlortetracycline fermentation solids | 15.4 | 64 |
| Sulfamethazine | 100 | 9 |
| Procaine Penicillin G | 60 | 8 |
| Calcium carbonate g.s. ad 100 | | 19 |
| Total | | 100 |

**0 041 114**

Example 3
Preparation of medicated granular feed premixes containing sulfamethazine
Three medicated feed premix batches of approximately 90 kg (200 lb) each are prepared, having the following composition:

| Component | kg | lb |
|---|---|---|
| Sulfamethazine | 18.1437 | 40 |
| Oyster shell meal | 36.2874 | 80 |
| Whey solids, sweet | 36.2874 | 80 |
| Total: | 90.7185 | 200 |

The components are mixed and blended for 10 minutes in a ribbon blender. The blended compositions are then fed to 10.16 cm (4") wide rolls of 25.4 cm (10") diameter at a predetermined pressure and rate to yield ribbons exceeding 30 kg Stokes hardness rating. The compacted samples are ground and sifted to 16/80 and 30/80 mesh fractions.

Example 4
Preparation of compacted, granular, sulfamethazine
A 20 g sample of sulfamethazine is compacted at 105.46 kg cm$^{-2}$ pressure. The compaction has an average (four measurements) Stokes hardness rating of 10.25 kg. The sample is ground and sifted to 60/100 mesh.

Example 5
Preparation of medicated granular feed premixes containing 50% by weight of sulfamethazine
Two medicated feed premix batches of 2,000 g each are prepared, having the following composition:

| Component | Wt in g | % by Wt of composition |
|---|---|---|
| Sulfamethazine | 1,000 | 50 |
| Calcium Carbonate | 500 | 25 |
| Whey solids, sweet | 500 | 25 |
| Total: | 2,000 | 100 |

The components are mixed and blended for 10 minutes in a double cone blender, and are then compacted at 105.46 kg cm$^{-2}$ pressure. The thus obtained compactions have an averaged (three measurements, each) Stokes hardness rating of 18.7 kg and 16.0 kg, respectively. The samples are ground and sifted to 0.59 mm/0.177 mm.

Example 6
Preparation of a medicated granular feed premix containing 10% by weight of sulfathiazole
A 2,000 g sample of medicated granular feed premix is prepared, having the following composition:

| Component | Wt in g | % by Wt of composition |
|---|---|---|
| Sulfathiazole | 200 | 10 |
| Whey solids, sweet | 1,000 | 50 |
| Calcium carbonate | 800 | 40 |
| Total: | 2,000 | 100 |

The components are mixed, blended, and are then compacted at 105.46 kg cm$^{-2}$ pressure. The compacted blend has a stokes hardness of 25.0 kg (average of three measurements). The sample is ground to 1.19 mm/0.250 mm.

7

**0 041 114**

Example 7

Determination of drug carry-over in a commercial feed mill during the preparation of medicated feed supplements

A total of 25 two-ton batches of medicated feed supplement crumbles are prepared using commercial equipment. Ten of these two-ton batches are prepared using standard medicated feed premixes while 15 of these two-ton batches are prepared from medicated feed premixes made by the method of the present invention.

The composition of the two-ton medicated feed supplement crumbles is as follows:

| Compound | Wt in lb | Wt in kg |
|---|---|---|
| Medicated feed premix* | 222.6 | 103.69 |
| Grain screenings | 1,800.0 | 816.47 |
| Alfalfa | 1,811.4 | 821.64 |
| Animal fat | 60.0 | 27.22 |
| Bond aid** | 100.0 | 45.36 |
| Totals: | 4,000.0 | 1,814.38 |

\*=composition of the medicated feed premixes is given in Table II.
\*\*=Sodium lignin sulfonate

TABLE II
Composition of medicated feed premixes

| Compound | Standard formulation | | | Granular, compacted formulation | | |
|---|---|---|---|---|---|---|
| | As is, in g | % by Wt | g/kg | As is in g | % by Wt | g/kg |
| Chlortetracycline fermentation solids (15.43%) | 226.8 | 50 | 77 | 226.8 | 50 | 77 |
| Sulfamethazine (100%) | 35 | 7.72 | 77 | 35 | 7.72 | 77 |
| Oil, mineral | 9.08 | 2 | 19.98 | — | — | — |
| Soybean meal, q.s. ad 100 | 182.71 | 40.28 | 401.99 | — | — | — |
| Whey solids, q.s. ad 100 | — | — | — | 191.79 | 42.28 | 421.94 |

The preparation of the ten two-ton batches of medicated feed supplement crumbles is followed by a clean-out procedure during which extensive samples are obtained. The clean-out procedure comprises a 90.8 kg "wash" and two two-ton "flushes". The "wash" and the two "flushes" consist of an alfalfa-grain screenings mixture with fat. The 90.8 kg "wash" is discarded while the two two-ton flushes usually are stored for later use in the preparation of similar medicated feed additives. A similar clean-out procedure follows the preparation of the 15 two-ton batches of medicated feed supplement crumbles prepared from the granular compacted premixes of the present invention.

After flushing, the feed mill is used to prepare two three-ton blends of non-medicated feed pellets, and the flushings following same, are analyzed for sulfamethazine contamination. The data obtained are summarized in Table III below wherein it can be clearly seen that preparations using the granular, compacted feed premixes of the present invention decrease the sulfamethazine contamination two to ten fold compared to similar standard medicated feed premixes.

8

**0 041 114**

TABLE III

Determination of drug carry-over in a commercial feed mill during the
preparation of feed supplements

| | | Sulfamethazine contamination in ppm | |
|---|---|---|---|
| Cumulative total kg of feed blended | Sample | Standard formulation | Granular, compacted formulation of the invention |
| 90.8 | 90.8 kg mixer flush | 337 | — |
| 1,906.8 | 1st two-ton flush of Mixing/conveying system | 25.2<br>37.0<br>40.7<br>39.2<br>35.4 | 215<br>6.5<br>3.2<br><3.2 |
| | Tail of 1st two-ton flush | 579 | 17.8 |
| 3,772.8 | 2nd two-ton flush of mixing system | 40.7<br>27.7<br>12.7<br>9.5 | 5.2<br>2.3<br><1.6<br>— |
| | Tail of 2nd two-ton flush | 11.8 | 6.6 |
| 6446.8 | 1st three-ton non-medicated through mixing/conveying system | No samples | 8.6<br>4.5<br>1.2 |
| | Tail of 1st three-ton batch | | 7.3 |
| 9170.8 | 2nd three-ton non-medicated through mixing/conveying system | 6.7<br>4.1<br>4.7<br>5.1<br>5.7 | 1.6<br>1.6<br>1.6<br>—<br>— |
| | Tail 2nd three-ton non-medicated blend | 80 | 8.0 |
| 10,986.8 | 1st two-ton flush of bagger and bagger bin | 35.8<br>14.2<br>6.9<br>3.7<br>4.0<br>24.5 | 21.8<br>16.1<br><1.5<br>3.0<br>20.5<br>1.4 |
| | Tail of 1st two-ton flush bagger | 80.0 | 3.0 |
| 12,802.8 | 2nd two-ton flush of bagger and bagger bin | 49.4<br>12.3<br>4.5<br>3.2<br>1.3 | 15.2<br>1.2<br>0.8<br>1.1<br><0.8 |
| | Tail of 2nd two-ton flush of bagger | — | 14.2 |
| 14,618.8 | 3rd two-ton through bagging bin | 2.1<br><0.5<br><0.5 | 7.6<br>0.3<br>0.5 |
| | Tail or 3rd two-ton lot | 5.1 | — |
| | 200 lb pellet mill flush | 192 | 127 |

9

Example 8

Determination of drug carry-over in a feed mill during the preparation of medicated feed supplements

Medicated pig feed supplements are prepared in four trials, each using a standard 18% protein swine starter formula in combination with:

a. Standard medicated premix;

b. Compacted granular medicated premix;

c. Compacted granular sulfamethazine (100%), prepared by the method of Example 4;

d. Compacted granular sulfamethazine (20% by weight of formulation), prepared by the method of Example 3.

Procedure

The drug premixes are incorporated each at a 11.35 kg per ton level in 4×454 kg batches, followed by one 454 kg non-medicated swine feed which serves as a flush. The composition of these rations are shown in Table IV.

Upon completion of the trial runs, each is followed by the preparation of 20×454 kg of non-medicated poultry layer formula (composition appended to Example) which is passed through the mill and is bagged.

Sampling

All samples are taken from bags prior to final closure. Samples are taken from the 8th and 16th bags of each medicated swine ration blend. Samples are taken from bags 1, 4, 8, 12, 16 and 20 of the non-medicated swine ration flush.

The poultry layer feed which follows the swine feed production is sampled as follows: batches 1 and 2 are sampled at bags 1, 4, 8, 12, 16 and 20, thereafter samples are taken only from bags 8 and 16.

The samples are analyzed and the amount of sulfamethazine present in the medicated and non-medicated feed supplements is determined. The results are shown in Table V, wherein it can be clearly seen that contamination of non-medicated feeds is markedly lower when compacted granular feed premixes or drugs are used, rather than the corresponding non-compacted standard formulations.

TABLE IV
Percent by weight composition of the medicated feed supplement of the example

| Component | Non-medicated | Medicated standard "a" | Medicated granular "b" | Sulfamethazine—granular | |
|---|---|---|---|---|---|
| | | | | "c" | "d" |
| Soybean meal (44%) | 83.5 | 82.25 | 82.25 | 83.442 | 83.21 |
| Dicalcium phosphate | 6.0 | 6.0 | 6.0 | 6.0 | 6.0 |
| Limestone | 6.5 | 6.5 | 6.5 | 6.5 | 6.5 |
| Salt | 2.5 | 2.5 | 2.5 | 2.5 | 2.5 |
| Trace Minerals* | 0.5 | 0.5 | 0.5 | 0.5 | 0.5 |
| Vitamin premix** | 1.0 | 1.0 | 1.0 | 1.0 | 1.0 |
| Standard premix | — | 1.25 | — | — | — |
| Granular premix | — | — | 1.25 | — | — |
| Granular sulfamethazine (100% by wt) | — | — | — | 0.058 | — |
| Granular sulfamethazine (20% by wt) "d" | — | — | — | — | 0.292 |

*=Analysis per kg:    Manganese, minimum 10%; Iron, minimum 10%; Zinc, minimum 10%; Calcium, maximum 5%, Calcium minimum 4%; Copper minimum 1%; Iodine minimum 0.3%; Cobalt, minimum 0.1%.

Ingredients:    Manganese sulfate, ferrous sulfate, ferrous carbonate, iron oxide, zinc sulfate, zinc oxide, calcium carbonate, copper oxide, potassium iodide, cobalt carbonate.

**=Analysis per kg:    Vitamin A 880,000 SI units; Vitamin $D_3$ 66,000 SI units; Vitamin E 4,400 I units, Riboflavin 990 mgs; Menadione ($K_3$) 341 mgs; d-Pantothenic acid 2,640 mgs; Niacin 5,500 mgs; Choline chloride 101,420 mgs; Vitamin $B_{12}$.

Ingredients:    Vitamin A acetate, d-activated animal sterol, dl-alpha tocopheryl acetate, menadione sodium bisulfite complex, riboflavin supplement, calcium pantothenate, niacin, choline chloride, Vitamin $B_{12}$ supplement, calcium carbonate roughage products, mineral oil and ethoxyquin, (a preservative).

TABLE V
Determination of drug carry-over in a feed mill during the
preparation of feed supplements

| Cumulative total kg of feed blended | Sample | ppm Sulfamethazine found in feed supplement | | | |
|---|---|---|---|---|---|
| | | a | b | c | d |
| 454 | Medicated Swine Conc. Batch 1, Bag 16 | 431 | 517 | 497 432 | 782 741 |
| 908 | Medicated Swine Conc. Batch 2, Bag 16 | 373 | 500 | ·467 480 | 674 584 |
| 6,362 | Medicated Swine Conc. Batch 3, Bag 16 | 356 | 514 | 379 508 · | 646 683 |
| 1,816 | Medicated Swine Conc. Batch 4, Bag 1 | | 500 | 327 | 469 |
| | Bag 4 | 298;301 | 607 | 402 | 464 |
| | Bag 12 | 398;419 | | 449 | 705 |
| | Bag 16 | 369;377 | | 576 | 753 |
| | Bag 20 | | 594 | 1,868 | 822 |
| 2,270 | Nonmedicated Swine Conc. Bag 1 | — | 18.1 | 11.4 | 6.8 |
| | Bag 4 | 11.3 | 2.4 | | |
| | Bag 8 | 13.7 | 6.5 | 12.6 | 7.4 |
| | Bag 12 | 16.1 | 2.3 | | |
| | Bag 16 | 17.7 | 4.9 | 12.8 | 15.7 |
| | Bag 20 | — | 49.5 | 190.0 | 61.0 |
| 2,724 | Poultry Layer Feed Batch 1, Bag 1 | 19.4 | 3.8 | 11.3 | 7.2 |
| | Bag 11 | — | 5.0 | | |
| | Bag 12 | 20.2 | — | 12.8 | 5.0 |
| | Bag 20 | 175 | 6.8 | 66.6 | 51 |
| 3,178 | Poultry Layer Feed Batch 2, Bag 1 | 8.9 | 2.4 | 3.6 | 3.5 |
| | Bag 12 | — | 1.5 | 4.1 | 3.4 |
| | Bag 20 | 94.6 | 5.2 | 47.5 | 28 |
| 3,632 | Poultry Layer Feed Batch 3, Bag 8 | 21.8 | 0.5 | 2.7 | 2.8 |
| | Bag 16 | 6.1 | 1.2 | 3.9 | 1.9 |
| 4,086 | Poultry Layer Feed Batch 4, Bag 8 | 3.4 | 0.5 | 1.6 | 1.2 |
| | Bag 16 | 3.5 | <0.5 | 2.1 | 1.3 |
| 4,540 | Poultry Layer Feed Batch 5, Bag 8 | 3.5 | <0.5 | 1.6 | 1.0 |
| | Bag 16 | 44.9 | <0.5 | 2.5 | 1.4 |
| 4,994 | Poultry Layer Feed Batch 6, Bag 8 | 3.0 | <0.5 | 0.8 | 0.6 |
| | Bag 16 | 2.0 | <0.5 | 0.6 | 0.6 |
| 5,448 | Poultry Layer Feed Batch 7, Bag 8 | 1.0 | <0.5 | 0.5 | 0.6 |
| | Bag 16 | 1.3 | <0.5 | 1.1 | 0.6 ·· |

| Cumulative total kg of feed blended | Sample | ppm Sulfamethazine found in feed supplement | | | |
|---|---|---|---|---|---|
| | | a | b | c | d |
| 5,902 | Poultry Layer Feed Batch 8, Bag 8 | 1.6 | <0.5 | 0.5 | 0.7 |
| | Bag 16 | 1.4 | <0.5 | 1.0 | 0.5 |
| 6,356 | Poultry Layer Feed Batch 9, Bag 8 | 0.7 | <0.5 | 0.5 | 0.6 |
| | Bag 16 | 0.8 | <0.5 | 0.7 | 0.7 |
| 6,810 | Poultry Layer Feed Batch 10, Bag 8 | 0.7 | <0.5 | 0.7 | 0.5 |
| | Bag 16 | 0.7 | 0.5 | 0.5 | 0.7 |
| 7,264 | Poultry Layer Feed Batch 11, Bag 8 | 0.7 | 2.2;2.8 | 0.7 | 0.6 |
| | Bag 16 | 0.6 | <0.5 | 0.5 | 0.6 |
| 7,718 | Poultry Layer Feed Batch 12, Bag 8 | 1.1 | <0.5 | 0.5 | 0.5 |
| | Bag 16 | 0.7 | <0.5 | <0.5 | 0.7 |
| 8,172 | Poultry Layer Feed Batch 13, Bag 8 | 0.5 | <0.5 | 0.5 | 0.7 |
| | Bag 16 | 0.8 | <0.5 | <0.5 | 0.5 |
| 8,626 | Poultry Layer Feed Batch 14, Bag 8 | 2.4 | <0.5 | <0.5 | 0.5 |
| | Bag 16 | 7.7 | <0.5 | 0.6 | 0.5 |
| 9,080 | Poultry Layer Feed Batch 15, Bag 8 | 173 | <0.5 | 0.9 | 0.5 |
| | Bag 16 | 2.9 | <0.5 | <0.5 | <0.5 |
| 9,534 | Poultry Layer Feed Batch 16, Bag 8 | 1.4 | <0.5 | <0.5 | <0.5 |
| | Bag 16 | 0.6 | <0.5 | 0.5 | 0.5 |
| 9,988 | Poultry Layer Feed Batch 17, Bag 8 | 0.5 | <0.5 | 0.5 | 0.5 |
| | Bag 16 | 0.6 | <0.5 | 0.5 | <0.5 |
| 10,442 | Poultry Layer Feed Batch 18, Bag 8 | <0.5 | <0.5 | <0.5 | 0.5 |
| | Bag 16 | 0.5 | <0.5 | <0.5 | 0.6 |
| 10,896 | Poultry Layer Feed Batch 19, Bag 8 | 0.6 | <0.5 | <0.5 | 0.5 |
| | Bag 16 | 0.6 | <0.5 | 0.6 | <0.5 |
| 11,350 | Poultry Layer Feed Batch 20, Bag 8 | 160 | <0.5 | 0.8 | <0.5 |
| | Bag 16 | 1.9 | <0.5 | 0.8 | <0.5 |

# 0 041 114

Poultry layer formula

| Component | | kg |
|---|---|---|
| Soybean meal | | 127.12 |
| Corn, ground | | 133.93 |
| Sorghum grain, ground | | 136.20 |
| Dehydrated alfalfa | | 11.35 |
| | Sub-total | 408.60 |
| Premix A (pounds) Dicalcium phosphate | | 11.35 |
| Limestone | | 13.62 |
| Salt | | 2.27 |
| Oyster shell (special pullet size) | | 13.62 |
| | Sub-total | 40.86 |
| Premix B (grams) Vitamin A (10,000 IU/g) | | 150 |
| Vitamin $D_3$ (15,000 IU/g) | | 60 |
| Vitamin $B_{12}$ (20 mg/lb) | | 120 |
| B-Complex (1233) | | 450 |
| D-L Methionine | | 350 |
| Trace minerals | | 230 |
| Corn, ground | | 3.180 |
| Sub-total (in kg) | | 4.54 |
| Total | | 454.0 |

## Claims

1. A process for preparing medicated animal feeds, feed additives, feed supplements or feed premixes, characterized by using in the process an antibiotic, a sulfa drug, penicillin, monensin, tylosin or lasalocid or a mixture of these drugs by either
(a) compacting the drug or mixture of drugs or
(b) compacting a blend of the drug or mixture of drugs blended with a compressible and pharmaceutically acceptable inert diluent or diluent mixture with sufficient force to obtain a

(1) compacted drug
(2) compacted mixture of drugs
(3) compacted blend of drug and diluent or diluents or
(4) compacted blend of a mixture of drugs and a diluent or diluents

of at least about 10 kg to 30 kg hardness on a Stokes hardness tester and grinding and sifting the compacted material to a particle size range of 2.0 to 0.099 mm to prevent excessive build-up, deposition, retention and dusting of the drug or mixture of drugs in the process.

2. A process according to Claim 1 comprising using in the process a sulfa drug and blending the compacted drug with a pharmaceutically acceptable inert diluent or diluent mixture in amounts sufficient to total the medicated animal feeds, feed additives, feed supplements or premixes to 100%.

3. A feed supplement or feed premix process according to Claim 2 comprising using sulfamethazine in

14

the process and blending the compacted sulfamethazine with other drugs and with a pharmaceutically acceptable inert diluent or diluent mixture in amounts sufficient to total the supplement or premix to 100%.

4. A process according to Claim 1 comprising using in the process a sulfa drug or a mixture of a sulfa drug and one or more of the additional drugs of Claim 1 by compacting a blend of sulfa drug or a mixture of a sulfa drug and one or more of the additional drugs of Claim 1 blended with a compressible and pharmaceutically acceptable inert diluent or diluent mixture.

5. A process according to Claim 4 wherein a mixture of sulfamethazine or sulfathiazole and at least one additional drug of tylosin, chlortetracycline or a stable penicillin is used in the compacted blend.

6. A process according to Claim 5 wherein the drug mixture comprises sulfamethazine, chlortetracycline and procaine penicillin G.

7. A process according to Claim 5 wherein the drug mixture comprises sulfamethazine or sulfathiazole blended with tylosin or salts thereof.

8. A process according to Claim 1 wherein the medicated animal feed supplement or premix preparation comprises: thoroughly blending 5% by weight to 65% by weight of the supplement or premix of a finely divided drug or a mixture of one or more finely divided drugs of tetracycline antibiotics, sulfa drugs or penicillin antibiotics with a compressible and pharmaceutically acceptable inert diluent or diluent mixture in amounts sufficient to total the supplement or premix to 100%; compacting the above blend with sufficient force to obtain compacted material of about 10 kg to about 30 kg hardness on a Stokes hardness tester; and grinding and sifting the compacted material to obtain a granular product of a particle size range of 2.0 to 0.99 mm which is sufficiently large to avoid segregation or electrostatic adhesion and sufficiently small to assure statistically uniform distribution of the drugs in the finished feed and which is sufficiently hard to resist shattering, abrasion or crumbling.

9. A process according to Claim 8 wherein the supplement or premix comprises: 16% by weight to 41% by weight of composition of chlortetracycline fermentation solids containing from 12% by weight to 27% by weight of chlortetracycline; 2% by weight to 6% by weight of composition of sulfamethazine; 2% by weight to 6% by weight of composition of procaine penicillin G; wherein the compacted material has a particle size range of 1.19 to 0.250 mm.

10. A process according to Claim 8 wherein the supplement or premix comprises: 32% by weight to 82% by weight of composition of chlortetracycline fermentation solids containing from 12% by weight to 27% by weight of chlortetracycline; 8% by weight to 13% by weight of composition of sulfamethazine; 7% by weight to 11% by weight of composition of procaine penicillin G; wherein the compacted material has a particle size range of 1.19 to 0.250 mm.

**Patentansprüche**

1. Verfahren zur Herstellung von medikamentiertem Tierfutter, Futteradditiven, Futterergänzungsstoffen oder Futter-Prämix, dadurch gekennzeichnet, daß man bei dem Verfahren ein Antibiotikum, eine Sulfa-Arznei, Penicillin, Monensin, Tylosin oder Lasalocid oder ein Gemisch dieser Arzneimittel verwendet, wobei man entweder

(a) die Arzneimittel oder ein Gemisch der Arzneimittel kompaktiert, oder

(b) ein Gemisch der Arzneimittel oder der Arzneimittelmischung vermischt mit einem komprimierbaren und pharmazeutisch akzeptablen inerten Verdünnungsmittel oder Verdünnungsmittelgemisch mit ausreichender Kraft kompaktiert, um

(1) ein kompaktiertes Arzneimittel
(2) ein kompaktiertes Arzneimittelgemisch
(3) eine kompaktierte Mischung von Arzneimittel und einen oder mehreren Verdünnungsmitteln oder
(4) ein kompaktiertes Gemisch einer Arzneimittelmischung und einem oder mehrerer Verdünnungsmittel
zu erhalten

mit einer Härte von mindestens etwa 10 kg bis 30 kg auf einem Stokes-Härtetestgerät, und das kompaktierte Material mahlt und siebt zu einem Teilchengrößenbereich von 2,0 bis 0,099 mm, um ein Übermaß an Anhäufung, Deposition, Retention und Staub des Arzneimittels oder der Arzneimittelmischung bei dem Verfahren zu verhindern.

2. Verfahren nach Anspruch 1, wobei man bei dem Verfahren ein Sulfa-Arzneimittel verwendet und das kompaktierte Arzneimittel mit einem pharmazeutisch akzeptablen inerten Verdünnungsmittel oder Verdünnungsmittel-Gemisch in ausreichenden Mengen vermischt, um die Gesamtmenge der medikamentierten Tierfutter, oder Futterzusatzstoffe, Futterergänzungsstoffe Prämixe auf 100% zu bringen.

3. Futterzusatzmittel- oder Futterprämix-Verfahren gemäß Anspruch 2, umfassend die Verwendung von Sulfamethazin bei dem Verfahren und das Vermischen des kompaktierten Sulfamethazins mit weiteren Arzneimitteln und mit einem pharmazeutisch akzeptablen inerten Verdünnungsmittel oder Verdünnungsmittelgemisch in ausreichenden Mengen, um die Gesamtmenge des Zusatzmittels oder Prämix auf 100% zu bringen.

4. Verfahren gemäß Anspruch 1, umfassend die Verwendung eines Sulfa-Arzneimittels oder einer

15

Mischung eines Sulfa-Arzneimittels und einem oder mehrerer der weiteren Arzneimittel von Anspruch 1 bei dem Verfahren, wobei ein Gemisch von Sulfa-Arzneimittel oder einer Mischung von einem Sulfa-Arzneimittel und einem oder mehrerer der weiteren Arzneimittel von Anspruch 1, gemischt mit einem komprimierbaren und pharmazeutisch akzeptablen inerten Verdünnungsmittel oder Verdünnungsmittelgemisch kompaktiert wird.

5. Verfahren gemäß Anspruch 4, wobei ein Gemisch von Sulfamethazin oder Sulfathiazol und mindestens einem weiteren der Arzneimittel Tylosin, Chlortetracyclin oder einem stabilen Penicillin in dem kompaktierten Gemisch verwendet wird.

6. Verfahren gemäß Anspruch 5, wobei das Arzneimittelgemisch Sulfamethazin, Chlortetracyclin und Procainpenicillin G enthält.

7. Verfahren gemäß Anspruch 5, wobei das Arzneimittelgemisch Sulfamethazin oder Sulfathiazol gemischt mit Tylosin oder Salzen desselben umfaßt.

8. Verfahren gemäß Anspruch 1, wobei die Herstellung des medikamentierten Tierfutterergänzungsstoffs oder Prämix folgendes umfaßt: das gründliche Vermischen von 5 Gew.-% bis 65 Gew.-%, bezogen auf den Ergänzungsstoff oder das Prämix, eines feinzerteilten Arzneimittels oder einer Mischung aus einem oder mehreren der feinzerteilten Arzneimittel Tetracyclinantibiotika, Sulfa-Arzneimittel oder Penicillinantibiotika mit einem komprimierbaren und pharmazeutisch akzeptablen inerten Verdünnungsmittel oder Verdünnungsmittelgemisch in ausreichenden Mengen, um die Gesamtmenge des Ergänzungsstoffs oder Prämix auf 100% zu bringen; die Kompaktierung der obigen Mischung mit ausreichender Kraft um ein kompaktiertes Material mit einer Härte von etwa 10 kg bis etwa 30 kg auf einem Stokes-Härtetestgerät zu erhalten; und das Mahlen und Sieben des kompaktierten Materials, um ein granulatförmiges Produkt mit einem Teilchengrößenbereich von 2,0 bis 0,099 mm zu erhalten, das ausreichend groß ist, um eine Segregation oder elektrische Adhäsion zu vermeiden und ausreichend klein, um eine befriedigend einförmige Verteilung der Arzneimittel in dem fertigen Futter zu gewährleisten und welches ausreichend hart ist, um gegen Schütteln, Abrieb oder Krümelung beständig zu sein.

9. Verfahren gemäß Anspruch 8, wobei das Ergänzungsmittel oder Prämix folgendes umfaßt: 16 Gew.-% der Zusammensetzung Chlortetracyclin-Fermentationsfeststoffe enthaltend von 12 Gew.-% bis 27 Gew.-% Chlortetracyclin, 2 Gew.-% bis 6 Gew.-% der Zusammensetzung Sulfamethazin; 2 Gew.-% bis 6 Gew.-% der Zusammensetzung Procainpenicillin G; wobei das kompaktierte Material einen Teilchengrößenbereich von 1,19 bis 0,250 mm aufweist.

10. Verfahren gemäß Anspruch 8, wobei das Ergänzungsmittel oder Prämix folgendes umfaßt: 32 Gew.-% bis 82 Gew.-% der Zusammensetzung Chlortetracyclin-Fermentationsfeststoffe, enthaltend von 12 Gew.-% bis 27 Gew.-% Chlortetracyclin; 8 Gew.-% bis 13 Gew.-% der Zusammensetzung Sulfamethazin; 7 Gew.-% bis 11 Gew.-% der Zusammensetzung Procainpenicillin G; wobei das kompaktierte Material einen Teilchengrößenbereich von 1,19 bis 0,250 mm aufweist.

**Revendications**

1. Procédé pour la préparation d'aliments, d'additifs alimentaires, de suppléments alimentaires ou de prémélanges alimentaires renfermant des médicaments pour animaux, caractérisé par l'emploi dans le procédé d'un antibiotique, d'un médicament de type sulfamide, de pénicilline, de monensine, de tylosine ou de lasalocide, ou d'un mélange de ces médicaments, soit par

(a) compactage du médicament ou d'un mélange de médicaments, soit

(b) compactage d'un mélange du médicament ou d'un mélange de médicaments mélangés avec un diluant ou un mélange diluant inertes compressibles et pharmaceutiquement acceptables avec une force suffisante pour obtenir

(1) un médicament compacté
(2) un mélange compacté de médicaments
(3) un mélange compacté de médicament et d'un ou plusieurs diluants, ou
(4) un mélange compacté d'un mélange de médicaments et d'un ou plusieurs diluants,

ayant une dureté d'au moins environ 10 kg à 30 kg avec un appareil d'essai de dureté Stockes et le broyage et le tamisage de la matière compactée à une taille de particules comprise dans la gamme de 2,0 à 0,099, pour éviter l'accumulation, le dépôt, la rétention et la pulvérisation excessifs du médicament ou du mélange des medicaments dans le procédé.

2. Procédé selon la revendication 1, comprenant l'emploi dans le procédé d'un médicament de type sulfamide, et le mélange du médicament compacté avec un diluant ou un mélange diluant inertes pharmaceutiquement acceptables en des quantités suffisantes pour que le total des aliments, additifs alimentaires, suppléments alimentaires ou prémélanges pour animaux, renfermant des médicaments, soit de 100%.

3. Procédé de préparation d'un supplément alimentaire ou d'un prémélange alimentaire selon la revendication 2, comprenant l'emploi de sulfaméthazine dans le procédé et le mélange de la sulfaméthazine compactée avec d'autres médicaments et avec un diluant ou un mélange diluant inertes pharmaceutiquement acceptables en des quantités suffisantes pour que le total du supplément ou du prémélange soit de 100%.

4. Procédé selon la revendication 1, comprenant l'emploi dans le procédé d'un médicament de type sulfamide ou d'un mélange d'un médicament de type sulfamide et d'un ou plusieurs des médicaments additionnels de la revendication 1, par compactage d'un mélange du médicament de type sulfamide ou d'un mélange d'un médicament de type sulfamide et d'un ou plusieurs des médicaments additionnels de la revendication 1, mélangé avec un diluant ou un mélange diluant inertes compressibles et pharmaceutiquement acceptables.

5. Procédé selon la revendication 4, dans lequel on utilise dans le mélange compacté un mélange de sulfaméthazine ou de sulfathiazole et d'au moins un médicament additionnel choisi parmi la tylosine, la chlortétracycline ou une pénicilline stable.

6. Procédé selon la revendication 5, dans lequel le mélange de médicaments comprend de la sulfaméthazine, de la chlortétracycline et de la procaïne-pénicilline G.

7. Procédé selon la revendication 5 dans lequel le mélange de médicaments comprend de la sulfaméthazine ou du sulfathiazole mélangés avec de la tylosine ou ses sels.

8. Procédé selon la revendication 1, dans lequel la préparation du supplément ou du prémélange alimentaire, renfermant des médicaments pour animaux, comprend: le mélange intime de 5% à 65% du poids du supplément ou du prémélange d'un médicament finement divisé ou d'un mélange d'un ou plusieurs médicaments finement divisés faisant partie des antibiotiques du groupe de la tétracycline, des médicaments de type sulfamide ou des antibiotiques du groupe de la pénicilline, avec un diluant ou un mélange diluant inertes, compressibles et pharmaceutiquement acceptables en des quantités suffisantes pour que le total du supplément ou du prémélange soit de 100%; le compactage du mélange ci-dessus avec une force suffisante pour obtenir une matière compactée ayant une dureté d'environ 10 kg à environ 30 kg avec un appareil d'essai de dureté Stockes; et le broyage et le tamisage de la matière compactée pour obtenir un produit granulaire ayant une gamme granulométrique de 2,0 à 0,099 mm, qui est suffisamment étendue pour éviter la ségrégation ou l'adhérence électrostatique et suffisamment étroite pour assurer une distribution statistiquement uniforme des médicaments dans l'aliment fini et qui est suffisamment dure pour résister à l'écrasement, à l'abrasion ou à l'émiettage.

9. Procédé selon la revendication 8, dans lequel le supplément ou prémélange comprend: 16% à 41% du poids de la composition de matières sèches de la production par fermentation de la chlortétracycline, contenant 12% en poids à 27% en poids de chlortétracycline; 2% à 6% du poids de la composition de sulfaméthazine; 2% à 6% du poids de la composition de procaïne-pénicilline G; dans lequel la matière compactée a une gamme granulométrique de 1,19 à 0,250 mm.

10. Procédé selon la revendication 8 dans lequel le supplément ou prémélange comprend: 32% à 82% du poids de la composition de matières sèches de la production par fermentation de la chlortétracycline contenant 12% en poids à 27% en poids de chlortétracycline; 8% à 13% du poids de la composition de sulfaméthazine; 7% à 11% du poids de la composition de procaïne-pénicilline G; dans lequel la matière compactée a une gamme granulométrique de 1,19 à 0,250 mm.